(19) Europäisches Patentamt
European Patent Office
Office européen des brevets

(11) Veröffentlichungsnummer: **0 043 550**
**B1**

# (12) EUROPÄISCHE PATENTSCHRIFT

(45) Veröffentlichungstag der Patentschrift: **29.06.83**

(51) Int. Cl.³: **C 12 Q 1/28**, G 01 N 33/52, C 12 N 9/96

(21) Anmeldenummer: **81105089.7**

(22) Anmeldetag: **01.07.81**

## (54) Mittel zum Nachweis peroxidatisch wirksamer Substanzen und Verwendung von Polyvinylmethylacylamid in einem solchen.

(30) Priorität: **04.07.80 DE 3025372**

(43) Veröffentlichungstag der Anmeldung: **13.01.82 Patentblatt 82/2**

(45) Bekanntmachung des Hinweises auf die Patenterteilung: **29.06.83 Patentblatt 83/26**

(84) Benannte Vertragsstaaten: **AT BE CH DE FR IT LI**

(56) Entgegenhaltungen:
**DE-A-1 930 059**
**DE-A-2 422 904**
**DE-A-2 546 252**
**DE-A-2 829 652**
**US-A-3 092 463**
**US-A-4 071 321**

(73) Patentinhaber: **BEHRINGWERKE AKTIENGESELLSCHAFT, Postfach 1140, D-3550 Marburg/Lahn (DE)**

(72) Erfinder: **Habenstein, Klaus, Dr., Birkenweg 3, D-3551 Lahntal (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

EP 0 043 550 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Mittel zum Nachweis peroxidatisch wirksamer Substanzen und Verwendung von Polyvinylmethylacylamid in einem solchen

Die Erfindung betrifft ein Mittel zur Bestimmung von peroxidatisch wirksamen Substanzen in Körperflüssigkeiten und Exkrementen, enthaltend ein Chromogen, ein Hydroperoxid, ein Detergenz, einen Aktivator und einen Stabilisator.

Der Nachweis peroxidatisch wirksamer Substanzen, zu denen im tierischen Organismus Hämoglobin und Myoglobin gehören, besitzt eine erhebliche Bedeutung bei der Ermittlung kleiner, mit dem Auge nicht sichtbarer Mengen Blut in Körperflüssigkeiten, wie Urin oder anderen Exkrementen, wie Stuhl oder Erbrochenem.

Entscheidend für die Aussagekraft des Blutnachweises ist bei einer Mikrohämaturie neben der schnellen Verfügbarkeit des Ergebnisses eine hohe Empfindlichkeit, Forderungen, die mit heute üblichen Testpapieren erfüllt werden können. Hierfür sind jedoch Chromogene von hoher Anzeigeempfindlichkeit und sehr kräftiger Farbentwicklung vonnöten. Zu den bekanntermassen hochempfindlichen Chromogenen gehören Benzidine, deren Anzeigeempfindlichkeit jedoch mit einer zum Teil äusserst geringen Lagerstabilität verbunden ist.

Zur Stabilisierung hochempfindlicher Chromogene wurde durch nacheinanderfolgende Mehrfachtränkungen eine räumliche Trennung zwischen Chromogenen und Hydroperoxid angestrebt. Zusätzlich wurde noch mit Stabilisatoren und stabilisierenden Lösungsmitteln gearbeitet. Es wurden auch schon filmbildende Schutzkolloide verwendet. Als solches ist Polyvinylpyrrolidin in der DE-A 2546252 beschrieben. Keine dieser Verfahrensweisen brachte jedoch bislang eine zufriedenstellende Verbesserung. Einen deutlich stabilisierenden Einfluss hat dagegen die Vewendung mikroverkapselter Hydroperoxide, die jedoch den Nachteil einer Reduktion der Anzeigeempfindlichkeit sowie einer ungleichmässigen und fleckigen Anzeige mit sich bringt.

Überraschend wurde nun gefunden, dass eine hohe Stabilisierung des Chromogens auf dem Testpapier durch Verwendung eines Polyvinylmethylacylamids der Formel I als Schutzkolloid

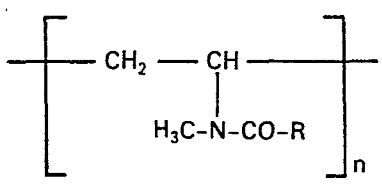

$n = 10^2$–$10^5$ I

$R = C_1$–$C_4$-Alkyl

erzielt wird.

Bevorzugte Bereiche sind:

$n = 10^3$–$10^4$;

$R = C_1$–$C_2$-Alkyl.

Gegenstand der Erfindung ist deshalb ein Mittel zur Bestimmung von peroxidatisch wirksamen Substanzen in Körperflüssigkeiten und Exkrementen, enthaltend ein Chromogen, ein Hydroperoxid, ein Detergenz, einen Aktivator und einen Stabilisator, dadurch gekennzeichnet, dass der Stabilisator aus einer Verbindung der Formel I besteht oder eine solche enthält.

Der Stabilisator kann bis zu 40%, bevorzugt bis zu 30%, eines anderen Schutzkolloids enthalten. Weiterhin kann der Stabilisator ein Copolymerisat mit einem Anteil von mindestens 60%, bevorzugt mindestens 70%, einer Verbindung der Formel I sein.

Geeignete Hydroperoxide sind beispielsweise Cumol-, Tetralin-, Dekalin- oder Pinan-hydroperoxide. Stabilisatoren vom Typ EDTA schützen das Hydroperoxid durch Abfangen von Schwermetallspuren. Statt der Benzidinderivate lassen sich unter anderem Guajakol oder heterocyclische Azine verwenden.

Als Detergenzien sind Substanzen vom Typ Natriumdodecylsulfat verwendbar.

Das Material des wasseraufnehmenden Testfeldes ist prinzipiell unkritisch. Verwendet werden allgemein Faservliese aus Cellulose oder Kunststoff. Es sind jedoch auch nicht-faserige Systeme bekannt, bei denen die Chemikalien in einen wasseraufnehmenden Film inkorporiert sind.

Falls das Mittel einen Puffer enthält, eignen sich als solche beispielsweise Citrate, Phosphate oder Phthalate, die nach Anfeuchten des Testfeldes einen pH-Wert von 4–7, vorzugsweise 5–6, erzeugen.

Zum Beweis der Überlegenheit des erfindungsgemässen Mittels gegenüber solchen des Standes der Technik wurden Teststreifen unter Verwendung eines der vorgeschlagenen Stabilisatoren beziehungsweise jeweils eines aus einer Auswahl von bekannten Stabilisatoren hergestellt und ihre Lagerstabilität verglichen. Die Abnahme der Anzeigeempfindlichkeit der bei 50°C gelagerten Teststreifen mit der Zeit diente als Mass für die Schutzwirkung der Stabilisatoren.

Es zeigte sich, dass die Anzeigeempfindlichkeit der erfindungsgemässen Teststreifen bedeutend weniger abnahm als die der Teststreifen mit bekannten Stabilisatoren.

Die Erfindung wird durch nachstehendes Beispiel näher erläutert.

Beispiel: Herstellung der Testpapiere

Etwa 100 $cm^2$ Indikatorrohpapier mit einem Flächengewicht von etwa 150 $g/m^2$ werden nacheinander mit folgenden Lösungen getränkt und jeweils zwischengetrocknet (Umlufttrockenschrank, 80°C):

1. Tränkung enthält
40 mg o-Tolidinhydrochlorid und 20 mg 4-Azafluoren in 10 ml Methanol;
2. Tränkung enthält
20 ml Natriumdodecylsulfat, 10 mg EDTA, 7,5 mg Tartrazin und 300 mg einer Verbindung der Formel I mit $n = 1400$ und $R = CH_3$, gelöst in 10 ml

eines 0,24 molaren Citronensäurepuffers, pH 5,5;
3. Tränkung enthält
250 mg Cumolhydroperoxid in 10 ml Frigen®.

Die fertigen Papiere werden in Dosen mit Trokkenmittel gelagert.

Durch Verwendung eines Produktes mit einem mittleren Molekulargewicht von $10^6$ wird ein Teststreifen ähnlicher Stabilität erhalten. Die Viskosität der 2. Tränklösung ist dann aber höher. Durch Zusatz hydrophobierender Copolymerer wird ein Polyvinylmethylacylamid erhalten, mit dem sich das hergestellte Testpapier in Richtung auf eine verbesserte Abstufbarkeit hin modifizieren lässt.

Eine gute Stabilisierung wird ebenfalls erreicht mit einer Mischung aus etwa 70% des obigen Produktes und etwa 30% eines anderen Filmbildners, beispielsweise eines Stärkederivates. Daraus ist ersichtlich, dass auch durch Zusatz des erfindungsgemässen Schutzkolloids zu an sich ungeeigneten Filmbildnern ein deutlicher Stabilisierungseffekt erzielt wird.

**Patentansprüche** für die Vertragsstaaten: BE, CH, LI, DE, FR, IT

1. Schnelldiagnostikum zum Nachweis peroxidatisch wirksamer Substanzen in Körperflüssigkeiten und Exkrementen, bestehend aus einer saugfähigen Trägermatrix, die ein Chromogen, ein Hydroperoxid, ein Detergenz, einen Aktivator und einen Stabilisator enthält, dadurch gekennzeichnet, dass der Stabilisator aus einer Verbindung der Formel I

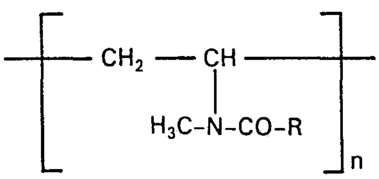

$n = 10^2-10^5$ I

$R = C_1-C_4$-Alkyl

besteht oder eine solche enthält.

2. Schnelldiagnostikum nach Anspruch 1, dadurch gekennzeichnet, dass $n = 10^3-10^4$ ist.

3. Schnelldiagnostikum nach Anspruch 1, dadurch gekennzeichnet, dass $R = C_1-C_2$-Alkyl ist.

4. Schnelldiagnostikum nach Anspruch 1, dadurch gekennzeichnet, dass der Stabilisator eine Verbindung der Formel I und bis zu 40% eines anderen Schutzkolloids enthält.

5. Schnelldiagnostikum nach Anspruch 1, dadurch gekennzeichnet, dass der Stabilisator eine Verbindung der Formel I und bis zu 30% eines anderen Schutzkolloids enthält.

6. Schnelldiagnostikum nach Anspruch 1, dadurch gekennzeichnet, dass der Stabilisator ein Copolymerisat mit einem Anteil von mindestens 60% einer Verbindung der Formel I ist.

7. Schnelldiagnostikum nach Anspruch 1, dadurch gekennzeichnet, dass der Stabilisator ein Copolymerisat mit einem Anteil von mindestens 70% einer Verbindung der Formel I ist.

8. Verfahren zum Nachweis peroxidatisch wirksamer Substanzen in Körperflüssigkeiten und Exkrementen, dadurch gekennzeichnet, dass ein Diagnostikum nach Anspruch 1 mit dem zu prüfenden Stoff zusammengebracht wird.

9. Verwendung einer Verbindung der Formel I gemäss Anspruch 1 in einem Diagnostikum.

**Patentansprüche** für den Vertragsstaat: AT

1. Verfahren zur Herstellung eines Schnelldiagnostikums zum Nachweis peroxidatisch wirksamer Substanzen in Körperflüssigkeiten und Exkrementen, dadurch gekennzeichnet, dass auf eine saugfähige Trägermatrix ein Chromogen, ein Hydroperoxid, ein Detergenz, ein Aktivator und als Stabilisator eine Verbindung der Formel I

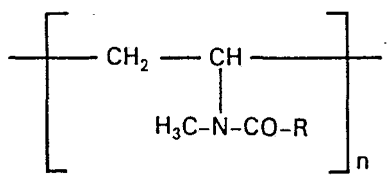

$n = 10^2-10^5$ I

$R = C_1-C_4$-Alkyl

aufgebracht wird.

2. Verfahren nach Anspruch 1, dadurch gekennzeichnet, dass als Stabilisator eine Verbindung der Formel I und bis zu 40% eines anderen Schutzkolloids aufgebracht wird.

3. Verfahren nach Anspruch 1, dadurch gekennzeichnet, dass als Stabilisator ein Copolymerisat mit einem Anteil von mindestens 60% einer Verbindung der Formel I aufgebracht wird.

4. Verfahren zum Nachweis peroxidatisch wirksamer Substanzen in Körperflüssigkeiten und Exkrementen, dadurch gekennzeichnet, dass ein Diagnostikum nach einem der Ansprüche 1 bis 3 mit dem zu prüfenden Stoff zusammengebracht wird.

**Claims** for the Contracting States: BE, CH, LI, DE, FR, IT

1. A rapid diagnostic agent for detecting, in body fluids and excreta, peroxidatively active substances, which comprises an absorbent carrier matrix containing a chromogen, a hydroperoxide, a detergent, an activator and a stabilizer, wherein the stabilizer comprises a compound of the formula I

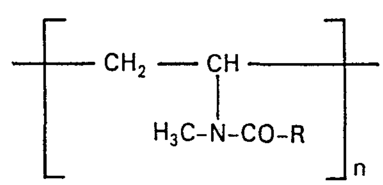

$n = 10^2-10^5$ I

$R = C_1-C_4$-alkyl

or contains such a compound.

2. A rapid diagnostic agent as claimed in claim 1, wherein n is $10^3-10^4$.

3. A rapid diagnostic agent as claimed in claim 1, wherein R is $C_1-C_2$ alkyl.

4. A rapid diagnostic agent as claimed in claim

1, wherein the stabilizer contains a compound of the formula I and up to 40% of another protective colloid.

5. A rapid diagnostic agent as claimed in claim 1, wherein the stabilizer contains a compound of the formula I and up to 30% of another protective colloid.

6. A rapid diagnostic agent as claimed in claim 1, wherein the stabilizer is a copolymer containing at least 60% of a compound of the formula I.

7. A rapid diagnostic agent as claimed in claim 1, wherein the stabilizer is a copolymer containing at least 70% of a compound of the formula I.

8. A method for detecting, in body fluids and excreta, peroxidatively active substances, which comprises bringing a diagnostic agent as claimed in claim 1 into contact with the material to be tested.

9. The use of a compound of the formula I according to claim 1 in a diagnostic agent.

**Claims** for the Contracting State: AT

1. A process for producing a diagnostic agent for detecting, in body fluids and excreta, peroxidatively active substances, which comprises that an absorbent carrier matrix is impregnated with a chromogen, a hydroperoxide, a detergent, an activator and a stabilizer, wherein the stabilizer is a compound of the formula I

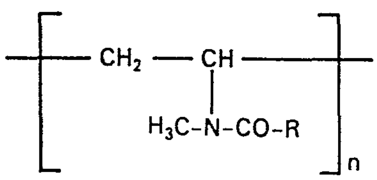

$n = 10^2–10^5$

I

$R = C_1$-$C_4$- alkyl

2. A process as claimed in claim 1, wherein the stabilizer contains a compound of the formula I and up to 40% of another protective colloid.

3. A process as claimed in claim 1, wherein the stabilizer is a copolymer containing at least 60% of a compound of the formula I.

4. A method for detecting, in body fluids and excreta, peroxidatively active substances, which comprises bringing a diagnostic agent according to one of the claims 1 to 3 into contact with the sample to be tested.

**Revendications** pour les Etats Contractants: BE, CH, LI, DE, FR, IT

1. Agent de diagnostic rapide pour la détection de substances à activité peroxydative dans des liquides corporels et des excréments, constitué d'un support absorbant contenant un chromogène, un hydroperoxyde, un détergent, un activateur et un stabilisant, caractérisé en ce que le stabilisant est constitué d'un composé de formule I

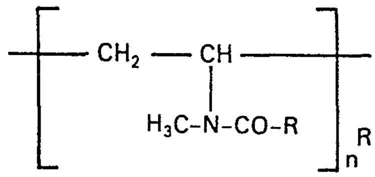

$n = 10^2$ à $10^5$

I

R = alkyle en $C_1$-$C_4$

ou contient un tel composé.

2. Agent de diagnostic rapide selon la revendication 1, caractérisé en ce que $n = 10^3$ à $10^4$.

3. Agent de diagnostic rapide selon la revendication 1, caractérisé en ce que R est un alkyle en $C_1$-$C_2$.

4. Agent de diagnostic rapide selon la revendication 1, caractérisé en ce que le stabilisant contient un composé de formule I et jusqu'à 40% d'un colloïde protecteur.

5. Agent de diagnostic rapide selon la revendication 1, caractérisé en ce que le stabilisant contient un composé de formule I et jusqu'à 30% d'un autre colloïde protecteur.

6. Agent de diagnostic rapide selon la revendication 1, caractérisé en ce que le stabilisant est un copolymère avec une proportion d'au moins 60% d'un composé de formule I.

7. Agent de diagnostic rapide selon la revendication 1, caractérisé en ce que le stabilisant est un copolymère avec une proportion d'au moins 70% d'un composé de formule I.

8. Procédé pour la détection de substances à activité peroxydative dans des liquides corporels et des excréments, caractérisé en ce que l'on met en contact un agent de diagnostic selon la revendication 1 avec le produit à examiner.

9. Utilisation d'un composé de formule I selon la revendication 1, dans un agent de diagnostic.

**Revendications** pour l'Etat Contractant: AT

1. Procédé de préparation d'un agent de diagnostic rapide pour la détection de substances à activité peroxydative dans les liquides corporels et les excréments, caractérisé en ce que l'on applique sur un support absorbant un chromogène, un hydroperoxyde, un détergent, un activateur et comme stabilisant un composé de formule I

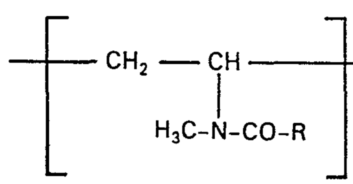

$n = 10^2$ à $10^5$

I

R = alkyle en $C_1$-$C_4$

2. Procédé selon la revendication 1, caractérisé en ce que l'on applique comme stabilisant un composé de formule I, et jusqu'à 40% d'un autre colloïde protecteur.

3. Procédé selon la revendication 1, caractérisé en ce que l'on applique comme stabilisant un copolymère avec une proportion d'au moins 60% d'un composé de formule I.

4. Procédé pour la détection de substances à

activité peroxydative dans les liquides corporels, et les excréments, caractérisé en ce que l'on met en contact un agent de diagnostic selon l'une des revendications 1 à 3 avec le produit à examiner.